(19) Europäisches Patentamt
European Patent Office
Office européen des brevets

(11) **EP 2 164 347 B1**

(12) **EUROPEAN PATENT SPECIFICATION**

(45) Date of publication and mention of the grant of the patent:
**29.06.2016 Bulletin 2016/26**

(21) Application number: **08774976.8**

(22) Date of filing: **10.07.2008**

(51) Int Cl.:
*A23L 27/23* (2016.01)     *A23L 33/10* (2016.01)
*C12N 1/06* (2006.01)     *A23L 13/40* (2016.01)

(86) International application number:
**PCT/EP2008/059013**

(87) International publication number:
**WO 2009/007424 (15.01.2009 Gazette 2009/03)**

(54) **YEAST AUTOLYSATES**

HEFEAUTOLYSATE

AUTOLYSATS DE LEVURE

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **10.07.2007 EP 07112167**

(43) Date of publication of application:
**24.03.2010 Bulletin 2010/12**

(73) Proprietor: **DSM IP Assets B.V.**
**6411 TE Heerlen (NL)**

(72) Inventors:
• **NOORDAM, Bertus**
**NL-2692 DA 's-Gravenzande (NL)**
• **KORTES, Jan, Gerrit**
**NL-3833 HV Leusden (NL)**

(74) Representative: **Kuster, Janaart Frans et al**
**DSM Intellectual Property**
**P.O. Box 4**
**6100 AA Echt (NL)**

(56) References cited:
**EP-A- 0 299 078**     **FR-A- 2 497 635**
**GB-A- 1 561 202**

• **JIAN ZHAO ET AL: "Degradation of RNA during the autolysis of Saccharomyces cerevisiae produces predominantly ribonucleotides" JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER-VERLAG, BE, vol. 32, no. 9, 1 September 2005 (2005-09-01), pages 415-423, XP019357760 ISSN: 1476-5535**

## Description

### Field of the invention

The present invention relates to yeast autolysates, to a process for producing yeast autolysates and to the use of yeast autolysates in food applications.

### Background of the invention

"Autolyzed yeast" or "yeast autolysate" has been known for many years as a source of protein, peptides, amino acids, fats, minerals and B-vitamins. The Food Chemical Codex defines Autolysed Yeast as follows: *"Autolysed Yeast is the concentrated, not extracted, partially soluble digest obtained from food-grade yeasts. Solubilisation is accomplished by enzyme hydrolysis or autolysis of yeast cells. Autolysed Yeast contains both soluble and insoluble components derived from the whole yeast cell"*.

A "yeast autolysate" is not the same as a "yeast extract" as can be deduced from th same Food Chemical Codex which defines a "yeast extract" as follows: *"Yeast Extract comprises the water soluble components of the yeast cell, the composition of which is primarily amino-acids, peptides, carbohydrates and salts. Yeast Extract is produced through the hydrolysis of peptide bonds by the naturally occurring enzymes present in edible yeast or by the addition of food-grade enzymes"*.

The yeast autolysate therefore differs from the "yeast extract" because the yeast autolysate, in addition to all the interesting components present in yeast extracts, also contains interesting components like $\beta$-glucans, mannoproteins and the yeast lipid fraction, present in the yeast cell wall. Another major difference is that the yeast autolysate contains a lot of insoluble components whereas the yeast extracts only comprise the water-soluble components of the yeast cell. A yeast extract contains more than 95% soluble material and usually up to 100%. In the production process of the yeast extract, the insoluble are removed by a suitable solid liquid separation whereas in the production of the yeast autolysate this step is lacking. The entire autolysate is subjected to a concentrating/drying step.

The presence of the high amount of insolubles in the prior art yeast autolysates is a severe disadvantage and limitation for their use in the food and/or feed industry: The high amount of insoluble solid matter results in rapid sedimentation when suspended in water.

For instance, a commercially available yeast autolysate BioSpringer BS2000 was found to contain approximately 60% insolubles (based on dry weight) which directly sediment when suspended in water. FR 2 497 635 also discloses a yeast autolysate.

The high amount of insolubles make the yeast autolysates unsuitable for modern applications such as the injection of flavour in for instance meat. In addition, their flavour profile is rather limited. On top of that, the low amount of solubilized protein, peptides and amino acids in classic yeast autolysates makes them less attractive for savoury food applications where the final flavour profile is amongst others dependent on for instance Maillard reactions (reactions in which the soluble fraction is involved).

Another disadvantage of prior art yeast autolysates is the almost complete absence or at least very low levels of 5'-GMP and 5'-IMP. This makes the prior art yeast autolysates unsuitable for applications where taste enhancement is desired. No commercially available yeast autolysate has been found which contains more than 1.5 wt% [5'-GMP + 5'-IMP] based on sodium chloride free dry matter (and wherein 5'-GMP and 5'-IMP are expressed as their $2Na.7H_2O$ salts). EP 0 299 078 discloses a yeast extract comprising 5'-GMP.

It is an object of the present invention to solve the problem associated with the prior art yeast autolysates and to provide yeast autolysates with a higher amount of solubles as well as a process for their production.

### Detailed description of the invention

#### Definitions

- Food Chemical Codex: Autolysed Yeast is the concentrated, not extracted, partially soluble digest obtained from food-grade yeasts. Solubilisation is accomplished by enzyme hydrolysis or autolysis of yeast cells. Autolysed Yeast contains both soluble and insoluble components derived from the whole yeast cell.
- Food Chemical Codex: Yeast Extract comprises the water soluble components of the yeast cell, the composition of which is primarily amino-acids, peptides, carbohydrates and salts. Yeast Extract is produced through the hydrolysis of peptide bonds by the naturally occurring enzymes present in edible yeast or by the addition of food-grade enzymes.
- The "dry solids ratio" is defined herein as the ratio between solubilised solids and total solids of the yeast autolysate.

The dry solids ratio is determined as follows: 5 grams of yeast autolysate is added to 100 grams of boiling water in a 200 ml glass beaker. The pH is approximately 6. The resulting suspension is stirred for 20 minutes with no extra heating. The experiment is carried out at room temperature and therefore the temperature of the suspension will slowly decrease. After 20 minutes and when the suspension has reached a temperature of between 20 and 70°C, 5 mL of the suspension is filtered over a 0.45 $\mu$m filter (0.45 $\mu$m GD/X PVDF syringe filter, 25 mm diameter, Whatman), and the permeate is collected. The dry solids in the permeate and the dry solids in the initial suspension are determined using a dry solids balance. Approximately 2 grams of the suspension is applied onto a filter paper, which is then applied to a Smart System[5] Moisture / Solids Analyzer Infra Red reader (CEM Matthews). The dry solids ratio is calculated as follows:

$$\text{Dry solids ratio} = \frac{\text{dry solids in the permeate (g)}}{\text{dry solids in the initial suspension (g)}} \times 100\%$$

- Yeast is defined herein as a solid, paste or liquid comprising yeast cells such as a fermentation broth used for the production of yeast or a cream yeast as used in the baking industry and which is a concentrated and washed suspension of yeast cells and which may have a yeast dry matter between 20-25% or even higher, or an active or instant dried yeast or any other form of product which comprises yeast cells.

In a first aspect the present invention provides a yeast autolysate having a dry solids ratio as defined hereinbefore of ≥50% and ≤95%. More preferably, the yeast autolysate has a dry solids ratio of ≥55%, more preferably ≥60%, more preferably ≥65%, more preferably ≥70%, more preferably ≥75%, more preferably ≥80%, more preferably ≥85 and ≤95% based on sodium chloride free dry matter of the yeast autolysate. Preferably, the yeast autolysate has a dry solids ratio of ≤90%. For a yeast autolysate containing sodium chloride, the value of the corresponding dry solid ratios will be higher due to the high solubility of sodium chloride. For instance, when a yeast autolysate without sodium chloride with a dry solids ratio of 50% is formulated with sodium chloride to a final sodium chloride concentration of 40 wt% (i.e. 60% yeast material), the resulting formulated yeast autolysate will have a dry solids ratio of 40 (NaCl) + 50% of 60% (yeast material) = 70%.

The yeast autolysate of the invention further comprises 5'-ribonucleotides. In the context of the present invention, "5'-ribonucleotides" refers to the total amount of 5'-monophosphate ribonucleotides formed during RNA degradation: 5'-monophosphate guanine (5'-GMP), 5'-monophosphate uracil (5'-UMP), 5'-monophosphate cytosine (5'-CMP), 5'-monophosphate adenine (5'-AMP), where 5'-AMP may be partially or completely converted into 5'-monophosphate inosine (5'-IMP). The yeast autolysate of the invention comprises at least 0.75% w/w 5'-GMP on sodium chloride free dry matter, more preferably at least 1% w/w 5'-GMP, more preferably at least 1.5% w/w 5'-GMP more preferably at least 2% w/w and most preferably, the yeast autolysate of the invention comprises at least 2.5% w/w 5'-GMP on sodium chloride free dry matter. Weight percentage calculations of the 5'-ribonucleotides are based on the disodium salt heptahydrate thereof unless otherwise specified. All percentages are calculated on sodium chloride free dry matter. In the present invention, the phrase "sodium chloride free dry matter" refers to the fact that for the calculation of the weight percentage the weight of any sodium chloride present is excluded from the yeast autolysate of the invention. The measurement of sodium chloride in the composition and the above-mentioned calculation can be performed by methods known to those skilled in the art.

Due to the constitution of RNA, 5'-UMP, 5'-CMP and 5'-AMP will also be present, but these nucleotides do not contribute significantly to taste or flavour enhancement. When 5'-AMP is transformed into 5'-IMP, typically by adenylic deaminase, the autolysate will comprises 5'-IMP, which contributes to flavour enhancement. Therefore, yeast autolysates containing 5'-IMP are also encompassed by the present invention. Preferably, the yeast autolysate of the invention comprises at least 0,75% w/w 5'-IMP on sodium chloride free dry matter, more preferably at least 1% w/w 5'-IMP, more preferably at least 1.5% w/w 5'-IMP, more preferably at least 2% w/w 5'-IMP and most preferably, the yeast autolysate of the invention comprises at least 2.5% w/w 5'-IMP on sodium chloride free dry matter. It will be understood by the skilled person, that it is highly preferred that the yeast autolysate of the invention comprises both 5'-GMP and 5'-IMP in the concentrations given above.

The yeast autolysate of the invention may further comprise salt, preferably sodium chloride. Preferably, the yeast autolysate of the invention comprises ≥ 5% and ≤ 50% w/w sodium chloride. More preferably, the yeast autolysate of the invention comprises at least 10%, more preferably at least 20%, more preferably at least 30%, more preferably between 35 and 45%, most preferably 40% - all w/w. Preferably, the yeast autolysate of the invention comprises ≤ 50% sodium chloride (w/w).

The yeast autolysate according to the present invention has as a major advantage in comparison with the prior art yeast autolysates that a greater fraction of the cell yeast constituents is solubilised. As a result thereof, a larger fraction of the cell constituents can contribute to flavour formation or enhancement in subsequent food applications. In fact, the functionality of the cell's constituents is utilised more efficiently. This is also reflected in the wider flavour profile which may be achieved with the autolysates according to the invention. A yeast autolysate of the present invention may therefore suitably be used in or as a carrier of top notes, in or as an ingredient for reaction flavour generation or in or as a flavour enhancer. The degree of solubilisation is so high that the autolysate of the present invention may advantageously be used in feed or food applications which require solubility higher than those of classic yeast autolysates. For example, the yeast autolysate of the present invention may suitably be used for meat injection, because it will not clog the injector. Solubilisation is reflected by the dry solids ratio in that a higher dry solids ratio results in a higher solubility. The higher the dry solids ratio, the higher the degree of solubilisation and the more peptides, amino acids and other compounds are available for participating in flavour generating reactions.

The yeast autolysate of the present invention differs from a yeast extract because it also contains valuable cell wall constituents, like beta-glucans, mannoproteins and the yeast lipid fraction. Beta glucans are known to have a positive effect on the immune response, while mannoproteins may contribute to Maillard reactions (and hence to flavour and taste). Similarly, lipids also positively influence the taste and/or flavour profile.

In a second aspect the invention provides a process for preparing the yeast autolysate of the invention having a dry solids ratio of ≥50t% and ≤95%, more preferably having a dry solids ratio of ≥55%, more preferably ≥60%, more preferably ≥65%, more preferably ≥70%, more preferably ≥75%, more preferably ≥80%, more preferably ≥85 and ≤95% based on sodium chloride free dry matter of the yeast autolysate, preferably ≤90% comprising:

a. subjecting yeast - as defined hereinbefore - to autolysis or hydrolysis at pH 4-7 and at a temperature in the range of 30-70°C in the presence of a protease; and
b. the reaction mixture obtained in step a) is subjected to further enzymatic treatment to produce 5'-ribonucleotides; and
c. concentrating the complete reaction mixture to yield the yeast autolysate,

with the proviso that the process does not comprise a solid liquid separation step in order to for instance separate insoluble cell walls from soluble cell components. The concentrated reaction mixture obtained after step c) is preferably dried by techniques known in the art.

In order to make the yeast autolysate of the invention comprising salt, preferably sodium chloride, the latter may be added during any step of the process but preferably before the drying step. For instance part of the salt may be added during step a) and the remainder before or after step c), or all may be added in step a) or before or after step c). The salt may be added as a solid, or in the form of a salt solution. The amount of salt to be added will depend on the required salt content of the yeast autolysate and may easily be determined by the skilled person.

In the process of the invention, the yeast which is subjected to autolysis or hydrolysis is preferably cream yeast as defined hereinbefore. In another embodiment of the invention, the yeast which is subjected to autolysis or hydrolysis is a yeast fermentation broth. The advantage of using the yeast fermentation broth as a starting material for the autolysis or hydrolysis instead if using cream yeast is an increased yield and a reduced cost price of the yeast autolysate since the intermediate concentrating and washing steps to obtain cream yeast can be omitted. Different starting materials will lead to yeast autolysates with a different taste profile.

The yeast may be any type of food-grade yeast, for example baker's yeast, beer yeast or wine yeast. Preferably, a yeast strain belonging to the genera *Saccharomyces, Kluyveromyces, Candida* or *Torula* is used. In a preferred embodiment a yeast strain belonging to the genus *Saccharomyces,* i.e. *Saccharomyces cerevisiae,* is used.

In one preferred embodiment, the yeast autolysate of the present invention is obtained by autolysis of yeast. In the context of the present invention, autolysis of yeast refers to a process well-known and described in the art, see e.g. Savory flavors, Tilak W. Nagodawithana, Esteekay Associates inc. 1995. Autolysis is the degradation of the yeast by its own endogenous enzymes after the yeast's cell wall has been damaged or disrupted. Damage or disruption of the cell wall may be effected by any suitable means, for example mechanically, chemically or enzymatically. A typical autolysate is rich in peptides and amino acids as compared to the total protein pool.

The process may be accelerated by the addition of exogenous enzymes. In one embodiment of the invention, autolysis is accelerated by the addition of one or more proteases, peptidases and/or glucanases.

In another preferred embodiment, the yeast autolysate of the present invention is obtained by hydrolysis of yeast. In the context of the present invention, hydrolysis of yeast refers to the process which is generally described as controlled lysis of the yeast cell. After inactivation of the yeast's endogenous enzymes, yeast degradation is performed by exogenously added enzymes. In one embodiment, one or more proteases, peptidases and/or glucanases are added to hydrolyse the yeast.

The protease or peptidase used for preparing the yeast autolysate may be of vegetable, bacterial or fungal origin and may be of distinct specificity, for example proline, cysteine, serine, glutamine or leucine specific. Suitable examples are included in EC 3.4.X and subclasses. Preferably, the protease is an endoprotease. More preferably the protease is a bacterial or a fungal endoprotease. The glucanase which may be used for preparing the yeast autolysate may also be of vegetable, bacterial or fungal origin, in particular those belonging to the class EC 3.2.1.6 enzymes.

In one embodiment, the yeast autolysate of the present invention is obtained using a combination of one or more proteases and/or peptidases and/or glucanases. In a preferred embodiment, an endoprotease and a glucanase are present to produce a yeast autolysate by autolysis or hydrolysis. The enzymes of the combination may be added simultaneously or consecutively. They may be added in one portion or in several portions. In another embodiment, the enzymatic treatment is combined with mechanical treatment.

The amount of enzyme to be added will depend on several factors, such as on the enzyme(s) which is (are) used and whether or not a combination with other treatments, such as mechanical treatment is applied. Typically, the one or more proteases, peptidases or glucanases are used in concentrations where the enzymes are in excess, which can easily be determined by the person skilled in the art. For economical reasons, the lowest amount of excess may be used.

The prior art processes producing the prior art yeast autolysates with low dry solids ratios (i.e. below 50%) are, for cost reasons, carried out in the shortest time course as possible, using the smallest amount of enzyme possible. A high dry solids ratio is not appreciated as a valuable property of the yeast autolysate. The yeast autolysates of the present invention may be obtained by the process of the invention. The difference between the process of the invention and the prior art processes resides in a higher degree of autolysis or hydrolysis of the yeast cell material. These higher degrees of autolysis or hydrolysis may be obtained by using more protease and/or by incubating for a longer time and/or at a higher temperature. Of course, the temperature should not be so high that it will damage the enzyme(s) used. The protease amounts required and optimal conditions in order to obtain the yeast autolysates of the present invention will be dependent on the combination of the type and the amount of enzyme used, the digestion time and the temperature. With routine optimisation, the skilled person can without undue burden determine the optimal conditions to obtain a dry solids ratio of at least 50% using a given enzyme and a given enzyme concentration. Alternatively, at a given temperature and/or maximum time of digestion, the skilled person can readily determine the amount of enzyme to be added in order to obtain the yeast autolysate of the invention. Suitable incubation times for step a) of the process of the invention may be from 1-50 hrs, preferably 2-40 hrs, more preferably 3-30 hrs, most preferably 5-20 hrs. A suitable pH for step a) of the process of the invention is a pH between 4 and 7, more preferably between 5 and 6, most preferably between 5.5 and 6.0. A suitable temperature for step a) of the process of the invention is between 30 and 70°C, more preferably between 40 and 60°C, most preferably between 45 and 55°C, e.g. 50°C.

In the process of the invention, prior to step c), the reaction mixture obtained in step a) is subjected to further enzymatic treatment to produce 5'-ribonucleotides and optionally an enzymatic treatment to convert 5'-AMP into 5'-IMP. In one embodiment, a 5'-ribonucleotide containing yeast autolysate is prepared by a process which comprises incubation with phosphodiesterase according to methods known in the art, preferably at a pH in the range of 4.8-5.5 and preferably at a temperature in the range of 55-68°C. Controlled hydrolysis allows for the production of yeast autolysates which contain 5'-ribonucleotides.

The yeast autolysate may be concentrated by methods known in the art, such as by reverse osmosis, ultrafiltration or evaporation. In a preferred embodiment, the yeast autolysate is concentrated by evaporation. After concentration, the yeast autolysate may be dried and formulated. In a preferred embodiment, the yeast autolysate is concentrated by evaporation and spray dried.

Using the process of the invention, yeast autolysates may be obtained with improved performance with regard to injectability, taste enhancement and flavour profile. Novel flavour profiles may be obtained depending on the yeast fraction which is used and depending on whether the yeast fraction has been subjected to autolysis or hydrolysis. Depending on the lysis method used, the yeast autolysate may comprise 5'-ribonucleotides in addition to the cell wall or cell wall components, proteins, peptides, amino acids, minerals, carbohydrates, B-vitamins.

In a third aspect, the present invention relates to the use of a yeast autolysate according to the invention in the feed, in particular the pet-food industry, or food industry, in particular the meat and meat processing industry. The products of the invention may be used as or in a flavour provider, e.g. in process flavour reactions, as or in a flavour enhancer, as or in a flavour improver, as or in a top note carrier. In one embodiment, the yeast autolysate is used in or as a tabletop application. A flavour enhancer, a flavour improver, a top-note carrier or a table-top application which comprises a yeast autolysate according to the invention is also encompassed in the present invention. The flavour enhancer, flavour improver, top-note carrier or table-top application may comprise 0.1-30% w/w of the autolysate of the present invention.

**EXAMPLES**

**Example 1**

**Generation of a yeast autolysate from cream yeast by autolysis**

One liter of cream yeast of *Saccharomyces cerevisiae* was heated to 50°C. Subsequently, 2 ml Alcalase® (Sigma Aldrich, containing protease from *Bacillus licheniformis*) was added and the mixture was incubated for 20 hours without pH adjustment. Solid sodium chloride was added so as to give a yeast autolysate with 40 wt% salt. Finally, the salt containing reaction mixture was concentrated by evaporation and spray-dried.

The resulting powder was analyzed for its composition and the results are presented in Table 1. The results demonstrate the very high amount of solubilised solids in the yeast autolysate. Sedimentation tests have demonstrated that it takes about 24 hours before sedimentation starts, while this only takes seconds to minutes in prior art yeast autolysates

Table 1 - Composition of the yeast autolysate of Example 1

| Component | % |
| --- | --- |
| dry solids ratio * | 86 |
| dry solids ratio *[1] | 76 |
| Nitrogen (Kjehldahl)[1] | 10.4 |
| Protein (N * 6.25)[1] | 65.0 |
| Amino nitrogen [1] | 3.6 |
| Amino nitrogen/total nitrogen (AN/TN) | 34.8 |
| Ash [1] | 6.0 |
| Solids (cell walls)[1] | 30.0 |
| Free amino acids /total amino acids | 37.1 |
| Glutamic acid[1] | 4.0 |
| * Based on filterability of a 5% suspension over a 0.45 $\mu$m filter. [1] on NaCl free dry matter | |

**Example 2**

**Generation of a yeast autolysate from cream yeast by hydrolysis**

One liter of cream yeast of *Saccharomyces cerevisiae* was heated at 95°C for 5 minutes and subsequently cooled down to 50°C. Next, 2 ml Alcalase® (Sigma Aldrich, containing protease from *Bacillus licheniformis*) was added and the mixture was incubated for 6 hours without pH adjustment. After this incubation, the mixture was treated with 5' phosphodiesterase for 15 hours at pH 5.3 and 65 °C to hydrolyse the RNA. After this, the mixture was treated with deaminase (Amano) for 5 hours at pH 5.1 and 55°C to convert the 5'-AMP into 5'-IMP. Solid sodium chloride was added so as to give a yeast autolysate with 40 wt% salt. Finally, the salt containing reaction mixture was concentrated by evaporation and spray-dried.

The resulting powder was analyzed for its composition and the results are presented in Table 2 which shows the very high amount of solubilised solids present in the yeast autolysate. The results in Table 2 also demonstrate the presence of more than 1.5% w/w 5' GMP and 5'IMP on the basis of NaCl free dry matter, which have never been reported before for yeast autolysates made by hydrolysis.

Table 2 - Composition of the yeast autolysate of Example 2

| Component | % |
| --- | --- |
| Dry solids ratio* | 80 |
| Dry solids ratio[1] | 67 |

(continued)

| Component | % |
|---|---|
| Nitrogen (Kjehldahl)[1] | 10.2 |
| Protein (N * 6.25)[1] | 63.8 |
| Amino Nitrogen[1] | 3.5 |
| Amino nitrogen/total nitrogen (AN/TN) | 34.3 |
| Ash[1] | 5.9 |
| Solids (cell walls)[1] | 32.1 |
| Free amino acids/total amino acids | 17.3 |
| 5'-GMP[1] | 2.6 |
| 5'-IMP[1] | 3.0 |
| Glutamic acid[1] | 2.1 |
| * Based on filterability of a 5% suspension over a 0.45 μm filter.<br>[1] on NaCl free dry matter | |

## Example 3

### Generation of a yeast autolysate by autolysis of a fermentation broth

One liter of fermentation broth of *Saccharomyces cerevisiae* was heated to 50°C. Subsequently, 1 ml Alcalase® (Sigma Aldrich, containing protease from *Bacillus licheniformis*) was added and the mixture was incubated for 20 hours without pH adjustment. Solid sodium chloride was added so as to give a yeast autolysate with 40 wt% salt. Finally, the salt containing reaction mixture was concentrated by evaporation and spray-dried.

The resulting powder was analysed for its composition and the results are presented in Table 3 and demonstrate the very high amount of solubilised solids in the yeast autolysate according to the invention (dry solids ratio 85%).

Table 3 Composition of the veast autolysate of Example 3

| Component | % |
|---|---|
| Dry solids ratio* | 85 |
| Dry solids ratio[1] | 75 |
| Nitrogen (Kjehldahl)[1] | 7.2 |
| Protein (N * 6.25)[1] | 45.0 |
| Amino nitrogen[1] | 2.4 |
| Amino nitroqen/total nitrogen (AN/TN) | 34.1 |
| Ash[1] | 21.5 |
| Solids (cell walls)[1] | 29.7 |
| Free amino acids/total amino acids | 37.4 |
| Glutamic acid[1] | 2.2 |
| Betaine[1] | 6.9 |
| * Based on filterability of a 5% suspension over a 0.45 μm filter.<br>[1] on NaCl free dry matter | |

EP 2 164 347 B1

### Example 4

### Comparison of dry solids ratios

The dry solids ratios of autolysates according to the invention were compared with the dry solids ratio of a commercial sample. Therefore 5 grams of each autolysate powder was suspended in boiling water and the total weight was made up to 100 g with water. Next, the different suspensions were filtered over a 0.45 $\mu$m filter. The dry solids ratio is a measure for the amounts of dissolved solids in the autolysate. The results are presented in Table 4.

Table 4 - Comparison of the solubility of different autolysates

| Yeast autolysate | Dry solids level in | | Dry solids ratio | |
|---|---|---|---|---|
| | suspension (%) | permeate (%) | autolysate as such (%) | Based on NaCl-free dry matter (%) |
| BioSpringer 2000 | 5.04 | 2.03 | 40 | 40 |
| Autolysate from Example 1 | 4.66 | 4.00 | 86 | 76 |
| Autolysate from Example 2 | 4.82 | 3.86 | 80 | 67 |
| Autolysate from Example 3 | 4.79 | 4.07 | 85 | 75 |

The results presented in Table 4 clearly demonstrate that the commercial yeast autolysate has lower levels of solubilised solids than the yeast autolysates according to the invention.

### Example 5

### Flavour profile of the new yeast autolysates

The autolysates from Examples 1, 2 and 3 were tested by a test panel. The results are presented in Table 5 and show that the flavour and taste of the autolysates prepared by the method of the invention varies depending on the starting material and the lysis method which is used. Flavour profiles ranges from yeasty to meaty and liquorice.

| | Taste | Smell |
|---|---|---|
| Autolysate example 1 | Yeasty, bouillon, slightly sulphuric | Sulphuric, yeasty |
| Autolysate example 2 | Sweet, full, slightly bouillon | Slightly yeasty |
| Autolysate example 3 | Meaty, yeasty, liquorice, sweet | Sweet, bouillon |

### Claims

1.   Yeast autolysate having a dry solids ratio of ≥50% and ≤95% based on sodium chloride free dry matter of the yeast autolysate and comprising at least 0.75% w/w 5'-GMP on sodium chloride free dry matter of the yeast autolysate, wherein the dry solids ratio is defined as the ratio between solubilised solids and total solids of the yeast autolysate.

2.   Yeast autolysate according to claim 1, having a dry solids ratio of ≥60% and ≤95% based on sodium chloride free dry matter of the yeast autolysate

3.   Yeast autolysate according to claim 1 or 2 which comprises at least 0.75% w/w 5'-IMP on sodium chloride free dry matter of the yeast autolysate.

4.   Yeast autolysate according to any of the preceding claims further comprising salt, preferably sodium chloride.

5.   A process for preparing the yeast autolysate as defined in any of claims 1-4 having a dry solids ratio of ≥50% and

8

≤95% comprising:

a. subjecting yeast to autolysis or hydrolysis at pH 4-7 and at a temperature in the range of 30-70°C in the presence of a protease;
b. the reaction mixture obtained in step a) is subjected to further enzymatic treatment to produce 5'-ribonucleotides; and
c. concentrating the complete reaction mixture to yield the yeast autolysate

with the proviso that the process does not comprise a solid liquid separation step.

6. The process according to claim 5 further comprising the step of drying the concentrated reaction mixture obtained in step b).

7. The process according to claim 5 or 6, further comprising adding salt, preferably sodium chloride.

8. Process according to any of claims 5-7, wherein the yeast is cream yeast or a fermentation broth.

9. Process according to anyone of claims 5-8, wherein the yeast is a species from the genera *Saccharomyces, Kluyveromyces, Candida or Torula,* preferably the genus Saccharomyces, more preferably *Saccharomyces cerevisiae*

10. Process according to any of claims 5-9 where the yeast is subjected to autolysis and the protease is an endogenous protease of the yeast

11. Process according to claim 10 comprising the addition of the addition of one or more proteases, peptidases and/or glucanases.

12. Process according to any of claims 5-11 where the yeast is subjected to hydrolysis and the protease is added after inactivation of the yeast endogenous enzymes.

13. Process according to claim 12 further comprising the addition of one or more proteases, peptidases and/or glucanases.

14. Process according to claim 10, wherein the further treatment comprises a further enzymatic treatment to convert 5'-AMP into 5'-IMP.

15. Use of a product obtained by a process according to anyone of claims 5-14 or a yeast autolysate according to anyone of claims 1-3 in a process flavour reaction, in meat applications, as or in a flavour enhancer, as or in a flavour improver, as or in a top note carrier or as or in a table-top application.

16. A flavour enhancer, a meat product, a flavour improver, a top-note carrier or a table-top application which comprises a yeast autolysate according to anyone of claims 1-4.


**Patentansprüche**

1. Hefeautolysat mit einem Trockenfeststoffverhältnis von ≥ 50 % und ≤ 95 % bezogen auf die natriumchloridfreie Trockenmasse des Hefeautolysats und umfassend wenigstens 0,75 % w/w 5'-GMP bezogen auf die natriumchloridfreie Trockenmasse des Hefeautolysats, wobei das Trockenfeststoffverhältnis als das Verhältnis zwischen solubilisierten Feststoffen und Gesamtfeststoffen des Hefeautolysats definiert ist.

2. Hefeautolysat gemäß Anspruch 1 mit einem Trockenfeststoffverhältnis von ≥ 60 % und ≤ 95 % bezogen auf die natriumchloridfreie Trockenmasse des Hefeautolysats.

3. Hefeautolysat gemäß Anspruch 1 oder 2, das wenigstens 0,75 % w/w 5'-IMP bezogen auf die natriumchloridfreie Trockenmasse des Hefeautolysats umfasst.

4. Hefeautolysat gemäß einem der vorstehenden Ansprüche, ferner umfassend Salz, vorzugsweise Natriumchlorid.

**5.** Verfahren zum Herstellen des Hefeautolysats gemäß einem der Ansprüche 1-4 mit einem Trockenfeststoffverhältnis von ≥ 50 % und ≤ 95 %, umfassend:

a. Unterwerfen von Hefe an Autolyse oder Hydrolyse bei einem pH-Wert von 4-7 und einer Temperatur in dem Bereich von 30-70 °C in Gegenwart einer Protease;
b. das bei Schritt a) erhaltene Reaktionsgemisch wird weiterer enzymatischer Behandlung unterzogen, um 5'-Ribonukleotide zu erhalten; und
c. Konzentrieren des vollständigen Reaktionsgemischs, um ein Hefeautolysat zu erhalten, mit der Maßgabe, dass das Verfahren keinen Feststoff-Flüssigkeit-Trennungsschritt umfasst.

**6.** Verfahren gemäß Anspruch 5, ferner umfassend den Schritt des Trocknens des bei Schritt b) erhaltenen konzentrierten Reaktionsgemischs.

**7.** Verfahren gemäß Anspruch 5 oder 6, ferner umfassend das Zugeben von Salz, vorzugsweise Natriumchlorid.

**8.** Verfahren gemäß einem der Ansprüche 5-7, wobei die Hefe Cremehefe oder eine Fermentationsbrühe ist.

**9.** Verfahren gemäß einem der Ansprüche 5-8, wobei die Hefe eine Spezies der Gattungen *Saccharomyces, Kluyveromyces, Candida* oder *Torula* ist, vorzugsweise der Gattung *Saccharomyces,* bevorzugter *Saccharomyces cerevisiae.*

**10.** Verfahren gemäß einem der Ansprüche 5-9, wobei die Hefe Autolyse unterworfen wird und die Protease eine endogene Protease der Hefe ist.

**11.** Verfahren gemäß Anspruch 10, umfassend das Zugeben von einer oder mehrerer Proteasen, Peptidasen und/oder Glucanasen.

**12.** Verfahren gemäß einem der Ansprüche 5-11, wobei die Hefe Hydrolyse unterworfen wird und die Protease nach Inaktivieren der Hefe-endogenen Enzyme zugegeben wird.

**13.** Verfahren gemäß Anspruch 12, ferner umfassend das Zugeben einer oder mehrerer Proteasen, Peptidasen und/oder Glucanasen.

**14.** Verfahren gemäß Anspruch 10, wobei die weitere Behandlung eine weitere enzymatische Behandlung zum Umwandeln von 5'-AMP zu 5'-IMP umfasst.

**15.** Verwendung eines Produkts, das durch ein Verfahren gemäß einem der Ansprüche 5-14 erhalten ist, oder eines Hefeautolysats gemäß einem der Ansprüche 1-3 bei einer Aromaverfahrensreaktion, bei Fleischanwendungen, als oder in einem Geschmacksverstärker, als oder in einem Geschmacksverbesserungsmittel, als oder in einem Kopfnotenträger oder als oder in Tafelanwendungen.

**16.** Geschmacksverstärker, Fleischprodukt, Geschmacksverbesserungsmittel, Kopfnotenträger oder Tafelanwendung, umfassend ein Hefeautolysat gemäß einem der Ansprüche 1-4.

**Revendications**

**1.** Autolysat de levure ayant un rapport de matière solide sèche ≥50% et ≤95% sur la base de la matière sèche dépourvue de chlorure de sodium de l'autolysat de levure et comprenant au moins 0,75% p/p de 5'-GMP sur la base de la matière sèche dépourvue de chlorure de sodium de l'autolysat de levure, dans lequel le rapport de matière solide sèche est défini comme le rapport entre les matières solides solubilisées et les matières solides totales de l'autolysat de levure.

**2.** Autolysat de levure selon la revendication 1, ayant un rapport de matière solide sèche ≥60% et ≤95% sur la base de la matière sèche dépourvue de chlorure de sodium de l'autolysat de levure.

**3.** Autolysat de levure selon la revendication 1 ou 2, comprenant au moins 0,75% p/p de 5'-IMP sur la base de la matière sèche dépourvue de chlorure de sodium de l'autolysat de levure.

**4.** Autolysat de levure selon l'une quelconque des revendications précédentes, comprenant en outre du sel, préférablement du chlorure de sodium.

**5.** Procédé de préparation de l'autolysat de levure tel que défini selon l'une quelconque des revendications 1-4, ayant un rapport de matière solide sèche ≥50% et ≤95%, comprenant les étapes consistant à :

a. soumettre la levure à une autolyse ou une hydrolyse à pH 4-7 et à une température dans la plage de 30-70°C en présence d'une protéase ;
b. le mélange réactionnel obtenu dans l'étape a) est soumis à un traitement enzymatique supplémentaire afin de produire des 5'-ribonucléotides ; et
c. concentrer le mélange réactionnel complet pour conduire à l'autolysat de levure, à condition que le procédé ne comprenne pas d'étape de séparation liquide-solide.

**6.** Procédé selon la revendication 5, comprenant en outre l'étape consistant à sécher le mélange réactionnel concentré obtenu dans l'étape b).

**7.** Procédé selon la revendication 5 ou 6, comprenant en outre l'addition de sel, préférablement de chlorure de sodium.

**8.** Procédé selon l'une quelconque des revendications 5-7, dans lequel la levure est de la crème de levure ou un bouillon de fermentation.

**9.** Procédé selon l'une quelconque des revendications 5-8, dans lequel la levure est une espèce issue du genre *Saccharomyces, Kluyveromyces, Candida* ou *Torula,* préférablement du genre *Saccharomyces,* plus préférablement *Saccharomyces* cerevisiae.

**10.** Procédé selon l'une quelconque des revendications 5-9, dans lequel la levure est soumise à une autolyse et la protéase est une protéase endogène de la levure.

**11.** Procédé selon la revendication 10, comprenant l'addition d'une ou plusieurs protéases, peptidases et/ou glucanases.

**12.** Procédé selon l'une quelconque des revendications 5-11, dans lequel la levure est soumise à une hydrolyse et la protéase est ajoutée après l'inactivation des enzymes endogènes de la levure.

**13.** Procédé selon la revendication 12, comprenant en outre l'addition d'une ou plusieurs protéases, peptidases et/ou glucanases.

**14.** Procédé selon la revendication 10, dans lequel le traitement supplémentaire comprend un traitement enzymatique supplémentaire afin de convertir le 5'-AMP en 5'-IMP.

**15.** Utilisation d'un produit obtenu par un procédé selon l'une quelconque des revendications 5-14 ou d'un autolysat de levure selon l'une quelconque des revendications 1-3, dans une réaction d'arôme de procédé, dans des applications de viande, comme ou dans un exhausteur d'arôme, comme ou dans un améliorateur d'arôme, comme ou dans un support de note de tête, comme ou dans une application de dessus de table.

**16.** Exhausteur d'arôme, produit carné, améliorateur d'arôme, support de note de tête, ou application de dessus de table, comprenant un autolysat de levure selon l'une quelconque des revendications 1-4.

**EP 2 164 347 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2497635

- EP 0299078 A